# EUROPEAN PATENT APPLICATION

(11) **EP 4 710 856 A1**
(43) Date of publication of application: **18.03.2026**
(21) Application number: 25200959.2
(22) Date of filing: 09.09.2025
(51) Int. Cl.: A61B 5/15, A61M 5/178

(54) **VISUAL INDICIA FOR ENHANCED SYRINGE VISIBILITY AND DETECTABILITY**

(30) Priority: 16.09.2024 US 202463695234 P
(71) Applicant: Burgess, Liam Isaac Emil, Saskatoon, Saskatchewan S7W0J6 (CA); Burgess, David Quentin, Saskatoon, Saskatchewan S7W0J6 (CA)
(72) Inventor: Burgess, Liam Isaac Emil, Saskatoon, Saskatchewan S7W0J6 (CA); Burgess, David Quentin, Saskatoon, Saskatchewan S7W0J6 (CA)
(74) Representative: Meissner Bolte Nürnberg

(57) **Abstract**

A syringe (10) having a barrel (12), and a plunger (14) retained within the barrel (12), the barrel (12) and/or plunger (14) provided with visual indicia (24, 26, 28, 30, 32) for increased visibility and detectability of the syringe (10) when spent and discarded, the visual indicia (24, 26, 28, 30, 32) being one or both of a non-natural design and a non-natural colour, the design and/or colour not obscuring any manufacturer or syringe content markings on the syringe (10). The visual indicia (24, 26, 28, 30, 32) may be either a pattern or a colour or both, where the pattern or colour present a visual contrast to a natural environment, such that the syringe (10) if discarded in the natural environment will be of increased visibility to persons seeking to detect the presence of the syringe (10). The visual indicia (24, 26, 28, 30, 32) may be applied to both the barrel (12) of the syringe (10) and the plunger (14) of the syringe (10). The visual indicia (24, 26, 28, 30, 32) may be applied to the barrel flange (16) or the plunger flange (18). The visual indicia (24, 26, 28, 30, 32) may be applied to a fully manufactured syringe (10) or incorporated within the syringe (10) during manufacture.

## Description

### TECHNICAL FIELD OF THE INVENTION

The present invention relates to syringe modifications, and more particularly to safety-related modifications of syringes.

### BACKGROUND OF THE INVENTION

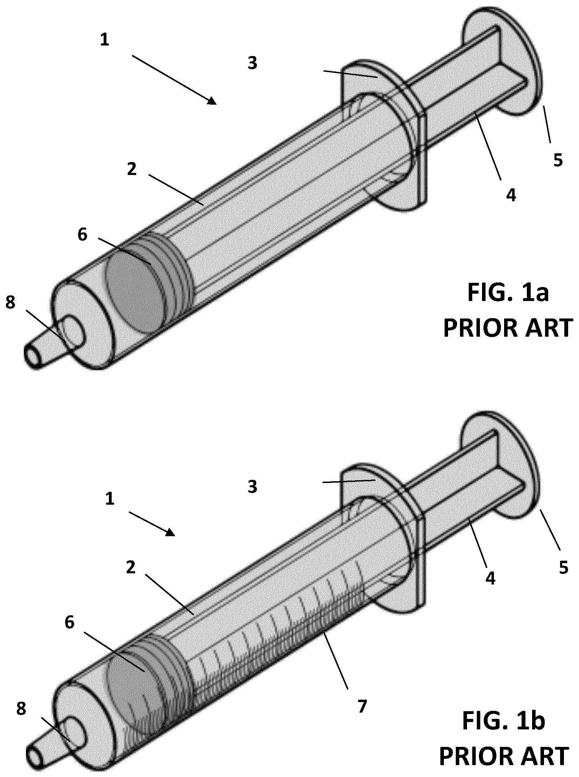

Syringes are ubiquitous in many settings, including without limitation health and protective services, research, and emergency response. FIG. 1a and 1b illustrate conventional prior art syringes 1, which syringes 1 comprise a barrel 2 retaining a reciprocating plunger 4 therein. The barrel 2 comprises a barrel flange 3 while the plunger 4 comprises a plunger flange 5, which flanges 3, 5 can be manipulated by a user to insert or withdraw the plunger 4 with respect to the barrel 2. The plunger 4 also comprises a seal 6 which travels within the barrel 2 interior, for discharging or withdrawing fluids based on unit markings 7 provided on the barrel 2; the barrel 2 is also commonly provided with content indicators, manufacturer brand indicators, and other visual indicia (not shown). The discharge or withdrawal of fluids occurs through a discharge orifice 8. The discharge orifice 8 is commonly provided with a needle or other sharp (not shown), often as part of an assembly that mates with the discharge orifice 8.

After a syringe is used, for example to inject medicine or withdraw blood, it is a "spent" syringe and is normally disposed of in a secure "sharps container" or similar receptacle in institutional settings such as hospitals. However, it is well known that use outside of such institutional settings can result in unsecured disposal, or even leaving such spent syringes lying on the ground. This has become a widespread problem in many countries, resulting both from legal self-medication use and illicit drug use.

Various persons find themselves in situations where they are tasked with locating and safely disposing of such spent syringes, for example custodial staff and civic employees. Other persons may be inadvertently exposed to them, such as teachers and students who come into contact with spent syringes in school grounds. In either case, the spent syringes are commonly composed of transparent plastic or glass and may therefore be difficult to detect in natural settings such as bushes, grass, or sand. Given that spent syringes may blend into their surroundings due in large part to visual transparency, there is an increased risk of inadvertent and accidental contact by a person with such spent syringes, which may result in needle punctures in the skin (so-called needlesticks) and potential transmission of pathogens.

Various solutions have been proposed in the prior art to address the risks associated with carelessly or inappropriately discarded syringes. For example, needle caps and self-neutralizing (or auto-disable) mechanisms have been designed to disable or fully and safely contain the sharp/needle components of spent syringes; however, needle caps are readily discarded or lost, and self-neutralizing (or auto-disable) mechanisms are not widely implemented due to cost considerations and the potential for malfunction.

Given that some prior art solutions are costly to implement, the significant per-unit cost increase renders such proposed solutions undesirable and negatively impacts their implementation. Costly solutions are more likely to be used in professional settings such as hospitals, but these are not the primary risk environments. Further, proposed solutions are generally for new syringes, without an option for retrofitting existing syringes in inventory.

What is needed, therefore, is a way to manufacture or modify a conventional syringe to render it more easily detectable, particularly in settings where a conventional syringe may visually blend into the surroundings which increases the risk of accidental needle punctures of a person's skin.

### SUMMARY OF THE INVENTION

The present invention is directed to increasing the visibility and detectability of syringes by applying or incorporating one or more types of visual indica to the syringe, the visual indicia selected so as to render the syringe more easily detected against a natural environment background, without the need for costly structural modifications to the conventional syringe, and where some embodiments may be implemented as a retrofit to existing syringe inventory.

The invention is set forth in the independent claims. Embodiments of the invention result from the dependent claims and the below description.

According to a first broad aspect of the present invention, there is provided a syringe comprising:
a barrel;
a plunger configured for retention within the barrel; and
at least one of the barrel and the plunger provided with visual indicia, the visual indicia comprising one or both of a non-natural design and an enhanced-visibility colour non-obscuring of any manufacturer or syringe content markings on the syringe;
such that when the syringe is spent and disposed of in a natural environment, the visual indicia provide a visual contrast to the natural environment to enhance visibility and detectability of the syringe.

The visual indicia may be either a pattern or a colour or both, where the pattern or colour present a visual contrast to a natural environment, such that the spent syringe if disposed of in the natural environment will be of increased visibility (to persons, cameras, and/or Al detection algorithms seeking to detect the presence of the spent syringe).

The visibility and detectability is preferably with respect to a nearby person, a camera, or an Al detection algorithm.

The visual indicia may be positioned non-overlapping to the manufacturer or syringe content markings on the syringe. The visual indicia may be semi-transparent or comprise a transparent portion and be overlapping with the manufacturer or syringe content markings on the syringe, so that the manufacturer or syringe content markings may be visible therethrough.

In some exemplary embodiments of the present invention, the visual indicia is applied to both the barrel of the syringe and the plunger of the syringe.

In some exemplary embodiments of the present invention, the barrel comprises a barrel flange and the visual indicia is applied to the barrel flange.

In some exemplary embodiments of the present invention, the plunger comprises a plunger flange and the visual indicia is applied to the plunger flange.

In some exemplary embodiments of the present invention, the visual indicia is applied to one or more external surfaces of the syringe after manufacture of the syringe. Such an application may, for non-limiting examples, be by adhesive film, heat-shrinkable tubing, etching, or printing applied to the one or more external surfaces.

In some exemplary embodiments of the present invention, the visual indicia may be inherent with the syringe and produced during manufacture of the syringe. For one non-limiting example, the visual indicia may be co-extruded with the syringe material at the time of manufacture.

In some exemplary embodiments, the non-natural design is repeating, whereas in some other exemplary embodiments the non-natural design is non-repeating.

The enhanced-visibility colour is preferably selected from the group consisting of a vivid translucent colour, a retro-reflective colour, and a fluorescent colour.

According to a second broad aspect of the present invention, there is provided a use of visual indicia on a spent syringe for enhanced visibility and detectability of the spent syringe in a natural environment, the visual indicia comprising one or both of a non-natural design and an enhanced-visibility colour non-obscuring of any manufacturer or syringe content markings on the spent syringe.

The visibility and detectability is preferably with respect to a nearby person, a camera, or an Al detection algorithm. The visual indicia may be semi-transparent or comprise a transparent portion and be overlapping with manufacturer or syringe content markings on the spent syringe, so that the manufacturer or syringe content markings are visible therethrough. The enhanced-visibility colour is preferably selected from the group consisting of a vivid translucent colour, a retro-reflective colour, and a fluorescent colour.

In some exemplary embodiments, at least one of the barrel and the plunger comprises a flange and the visual indicia is applied to the flange.

According to a third broad aspect of the present invention, there is provided a method for enhancing visibility and detectability of a syringe in a natural environment, the method comprising the steps of:
a. assessing the natural environment to identify at least one of a natural colour and a natural pattern present in the natural environment;
b. determining at least one non-natural visual indicia;
c. selecting at least one of the non-natural visual indicia as presenting a visual contrast to the natural colour or the natural pattern; and
d. applying the selected non-natural visual indicia to the syringe;
such that when the syringe is present in the natural environment, the syringe has enhanced visibility and detectability compared to the surrounding natural environment.

In some exemplary embodiments, the non-natural visual indicia comprises at least one of a non-natural colour and a non-natural pattern. The enhanced visibility and detectability is preferably with respect to a nearby person, a camera, or an Al detection algorithm. In some exemplary embodiments, the non-natural colour is selected from the group consisting of a vivid translucent colour, a retro-reflective colour, and a fluorescent colour.

Embodiments of the present invention may therefore provide syringes, and particularly spent syringes, that are modified so as to present a visual contrast to the surrounding natural environment in which the syringe is discarded. It is known in the art of syringe manufacture to apply patterns or colours to syringes for various purposes, such as for example providing a stress-reduction effect (United States Patent Application Publication No. 2005/0209567 to Sibbitt, Jr.), using colours to indicate different dosing ranges (United States Patent No. 6,764,469 to Broselow; United States Patent Application Publication No. 2023/0405232 to Hernandez), and using different syringe plunger colours to identify different medications (United States Patent Application Publication No. 2006/0084925 to Ramsahoye), but the prior fails to teach or even suggest selecting visual indicia in order to provide a visual contrast to a natural environment to render the syringe more readily detectable in order to enable its removal and prevent injury or illness. Further, the prior art is wholly directed to using visual modifications in order to enhance use of the syringe, not to enhance visibility and detectability after the syringe is spent and discarded.

A detailed description of exemplary embodiments of the present invention is given in the following. It is to be understood, however, that the invention is not to be construed as being limited to these embodiments. The exemplary embodiments are directed to particular applications of the present invention, while it will be clear to those skilled in the art that the present invention has applicability beyond the exemplary embodiments set forth herein.

### BRIEF DESCRIPTION OF THE DRAWINGS

In the accompanying drawings, which illustrate exemplary embodiments of the present invention:
FIG. 1a is a perspective view of a prior art syringe without unit markings.
FIG. 1b is a perspective view of a prior art syringe with unit markings.
FIG. 2 is a perspective view of a first embodiment of the present invention, with a non-natural design in a non-natural colour applied to a barrel of the syringe.
FIG. 3a is a perspective view of a second embodiment of the present invention, with a non-natural design in a non-natural colour applied to both the barrel and the plunger of the syringe, the syringe without unit markings.
FIG. 3b is a perspective view of the second embodiment of the present invention, with a non-natural design in a non-natural colour applied to both the barrel and the plunger of the syringe, the syringe with unit markings that are not obscured by the semi-transparent design.
FIG. 4 is a perspective view of a third embodiment of the present invention, with a non-natural design in a non-natural colour applied to only the part of the syringe barrel without unit markings.
FIG. 5 is a perspective view of a fourth embodiment of the present invention, with a non-natural design in a non-natural colour applied to the plunger of the syringe and only the part of the syringe barrel without unit markings.
FIG. 6 is a perspective view of a fifth embodiment of the present invention, with a non-natural design in a non-natural colour applied to the plunger of the syringe and only the part of the syringe barrel without unit markings, and the non-natural colour is also applied to the plunger flange.
FIG. 7 is a perspective view of a sixth embodiment of the present invention, with a non-natural design in a non-natural colour applied to the plunger of the syringe and only the part of the syringe barrel without unit markings, and the non-natural colour is also applied to the barrel flange.
FIG. 8 is a perspective view of a seventh embodiment of the present invention, with a non-natural design in a non-natural colour applied to the plunger of the syringe and only the part of the syringe barrel without unit markings, and the non-natural colour is also applied to the barrel flange and the plunger flange.
FIG. 9 is a flowchart illustrating a method for applying or inhering a design according to the present invention to a syringe.

Exemplary embodiments will now be described with reference to the accompanying drawings.

### DETAILED DESCRIPTION OF EXEMPLARY EMBODIMENTS

Throughout the following description, specific details are set forth in order to provide a more thorough understanding to persons skilled in the art. However, well known elements may not have been shown or described in detail to avoid unnecessarily obscuring the disclosure. The following description of examples of the invention is not intended to be exhaustive or to limit the invention to the precise form of any exemplary embodiment. Accordingly, the description and drawings are to be regarded in an illustrative, rather than a restrictive, sense.

The present invention is directed to syringe modifications to enhance visibility and detectability of spent syringes that have been improperly disposed of, for example in a natural environment such as bushes, grass, or sand. Conventional syringes are composed of transparent base material, such as plastic or glass, and they accordingly blend in with their surroundings, increasing the risk of needlestick and potential pathogen transmission. Where a spent syringe has been discarded in a natural environment, non-natural visual indicia such as designs and colours may be more readily noticed and detected (by nearby persons, cameras, Al detection algorithms, and the like), enabling more cautious and safety-conscious handling of the spent syringe.

The visual indicia may be a non-natural visual pattern, such as for one non-limiting example a repeating regular pattern or a non-repeating pattern, which could be more noticeable to persons when the spent syringe is discarded in a natural environment. Alternatively or additionally, the visual indicia may be an enhanced-visibility colour scheme comprising one or more bright, vibrant, saturated colours, even a visually jarring colour, which again is more noticeable to persons. The colour scheme, for some non-limiting examples, may be a vivid translucent colour, retro-reflective colour, or fluorescent colour. As noted above, the problem is that syringes are commonly manufactured from clear, transparent materials that blend into whatever environment they are discarded in. By applying or incorporating a design or colour that stands out against the surrounding physical environment, the syringe may be rendered more detectable and thus reduce the risk of needlestick.

Further, the visual indicia should be applied to or incorporated in the syringe in such a way that the syringe functionality is not negatively impacted. For example, the visual indicia may be semi-transparent, allowing the user to see the markings on the barrel such as unit markings or syringe content indicators. Alternatively, the visual indicia may be applied to or incorporated in the syringe on a portion of the barrel not provided with markings such as unit markings or syringe content indicators.

The visual indicia may be applied to a barrel exterior surface by adhesive film, heat-shrinkable tube, silk screening, or analogous printing means, which application methods are known to those skilled in the art. The visual indicia may in some embodiments be applied to the syringe by etching or an analogous technique know to those skilled in the art. The visual indicia may be integrated into the syringe during manufacture thereof, for example through extrusion, mold injection, 3D printing, or similar methods, at a suitable point in the manufacturing process as would be identifiable by one skilled in the art.

The visual indicia may be applied to the syringe exterior to varying degrees. For one non-limiting example, in some exemplary embodiments the visual indicia covers at least 40% of the barrel exterior surface area, to optimize detectability. However, the visual indicia may cover a significantly smaller area so long as it enhances visibility.

In embodiments where the visual indicia is applied to the barrel exterior surface after manufacture of the syringe, it will be clear that the visual indicia may be applied to syringes of varying length and diameter, enhancing ease of widespread implementation. Further, by relying on the application or incorporation of visual indicia rather than a structural modification, additional training for users should not be required.

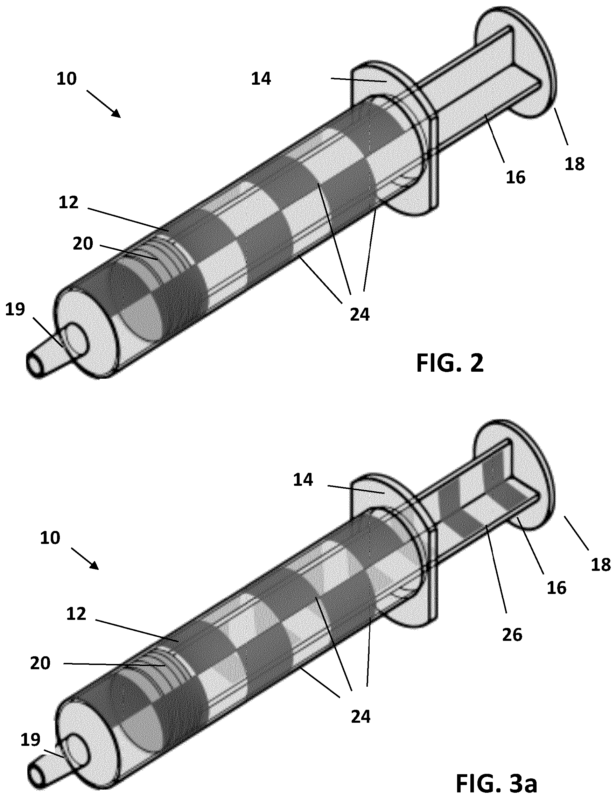

Turning now to the accompanying drawings, FIG. 2 illustrates in perspective view a first exemplary embodiment of the present invention. The illustrated syringe 10 comprises a barrel 12 and a reciprocating plunger 16 retained therein, the plunger 16 configured to move axially between withdrawn and inserted positions within the barrel 12. The barrel 12 comprises a barrel flange 14, and the plunger 16 comprises a plunger flange 18, such that a user can engage the barrel flange 14 with their fingers and the plunger flange 18 with their thumb to axially move the plunger 16 relative to the barrel 12. The syringe 10 further comprises a conventional discharge orifice 19, a seat for the needle assembly (now shown) used in ejection (e.g., medicine) or intake (e.g., blood) of fluid, a seal 20 on the plunger 16 configured to engage the barrel 12 interior wall to force fluid out of the barrel 12 or help create a vacuum to draw fluid into the barrel 12.

FIG. 2 illustrates a first exemplary form of visual indicia, specifically a design 24 which is applied to the entire outer surface of the barrel 12. The design 24 is a repeating pattern in the exemplary form of a checkerboard. While illustrated in a bright colour that itself could be a visual indicia, the design 24 need not be presented in a bright colour - the regular repeating pattern alone may be sufficient to enhance visibility and detectability of the syringe 10 once spent and discarded. As noted above, the design 24 may be applied post-manufacturing, such as by an adhesive film, a heat-shrinkable tube, silk screening, or analogous printing means, or by etching into the barrel 12 surface. Alternatively, the design 24 may be incorporated into the barrel 12 during manufacture. As can be seen from FIG. 2, the design 24 is semi-transparent, allowing a user to see through the design 24 when initially operating the syringe 10.

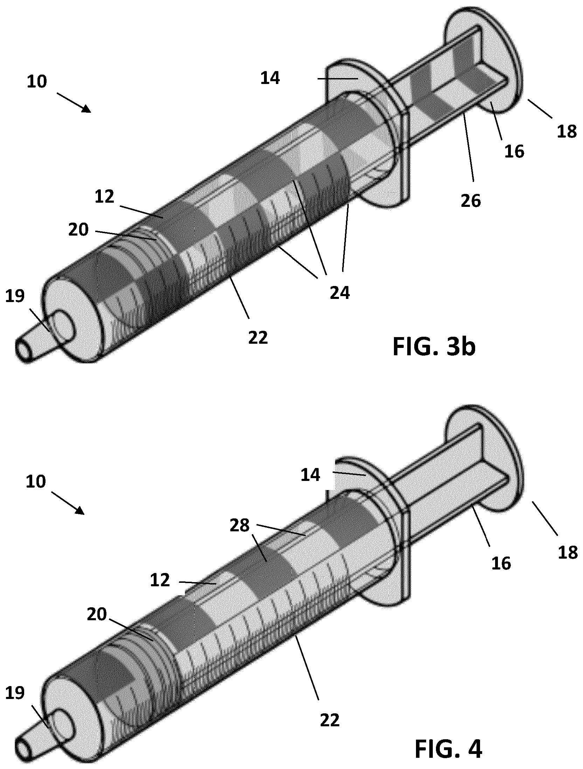

Turning now to FIG. 3a and 3b, the syringe 10 comprises the barrel 12 and the plunger 16, and both the barrel 12 and the plunger 16 are provided with visual indicia, specifically the full-barrel design 24 on the barrel 12 (as in FIG. 2) and a repeating design 26 on the plunger 16. Whether the plunger 16 is inserted or withdrawn, the plunger design 26 will be visible given the transparent syringe 10 material and the semi-transparent barrel design 24. Again, while shown in a vibrant colour, the plunger design 26 need not be presented in a vibrant colour as the pattern of the design 26 may enhance visibility and detectability.

FIG. 3b shows the same embodiment as FIG. 3a, but with conventional unit indicators 22 provided on the side of the barrel 12. It is well known that such unit indicators 22 represent volume indicators and help users to accurately determine a suitable amount of fluid to inject (e.g., medicine) or withdraw (e.g., blood). It can be seen in FIG. 3b that the unit indicators 22 are still visible despite the overlapping presence of the barrel design 24, the presence of the design 24 therefore not negatively impacting the use of the syringe 10.

FIG. 4, in contrast, presents an embodiment where the visual indicia is provided on the barrel 12 but not overlapping with the unit indicators 22. A half-barrel design 28 is provided on the barrel 12, on the half of the barrel 12 not provided with the unit indicators 22. This could allow semi-transparent or opaque visual indicia, as they would not obscure the unit indicators 22 in any way.

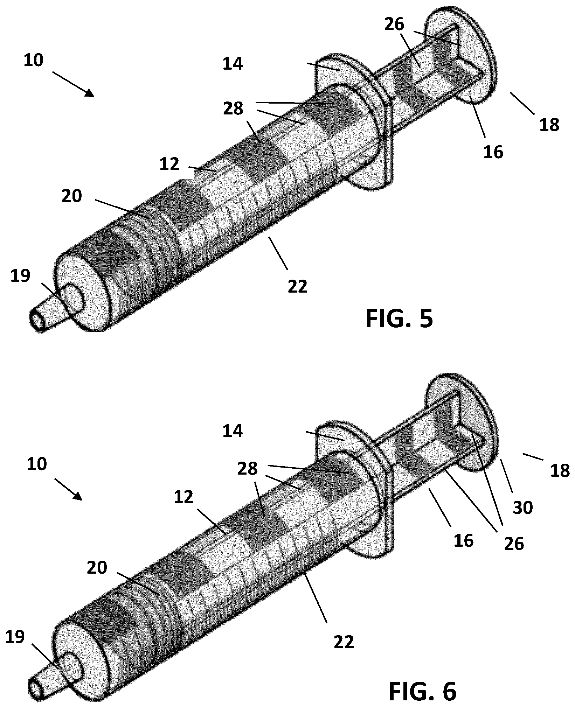

FIG. 5 illustrates yet another exemplary embodiment, in this case where the visual indicia is present on two components of the syringe 10. Unlike FIG. 3b which illustrates the syringe 10 provided with the full-barrel design 24 and the plunger design 26, FIG. 5 illustrates an embodiment comprising the half-barrel design 28 and the plunger design 26.

FIG. 6 to 8 illustrate further exemplary embodiments where the flanges 14, 18 are also used in enhancing the visibility and detectability of the syringe 10. In FIG. 6, the barrel 12 is provided with the half-barrel design 28 and the plunger 16 is provided with the plunger design 26, but the plunger flange 18 is also provided with plunger flange indicia 30, which in the illustrated embodiment is a vibrant colour rather than a design. It should be noted that while a colour is used for the indicia 30, a design could be alternatively or additionally used on the plunger flange 18.

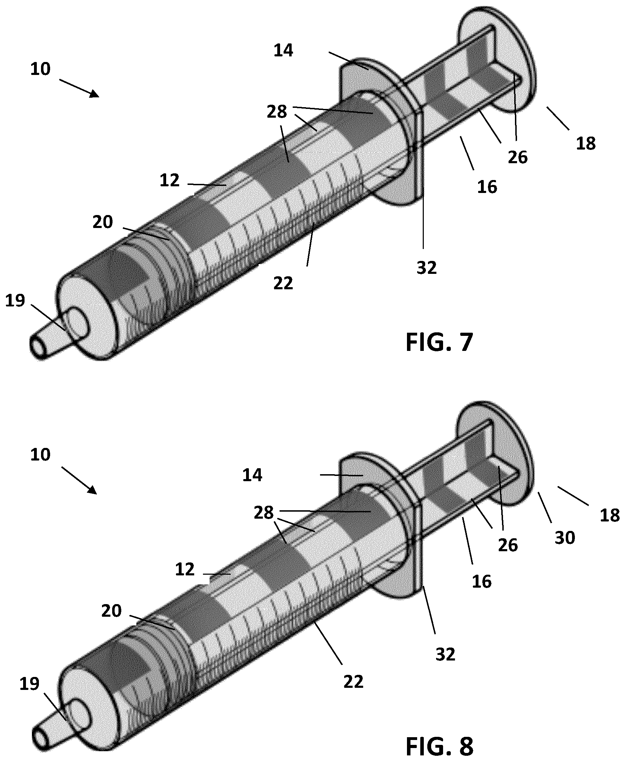

In FIG. 7, the barrel 12 is provided with the half-barrel design 28 and the plunger 16 is provided with the plunger design 26, but the barrel flange 14 is also provided with barrel flange indicia 32, which in the illustrated embodiment is a vibrant colour rather than a design. It should be noted that while a colour is used for the indicia 32, a design could be alternatively or additionally used on the barrel flange 14.

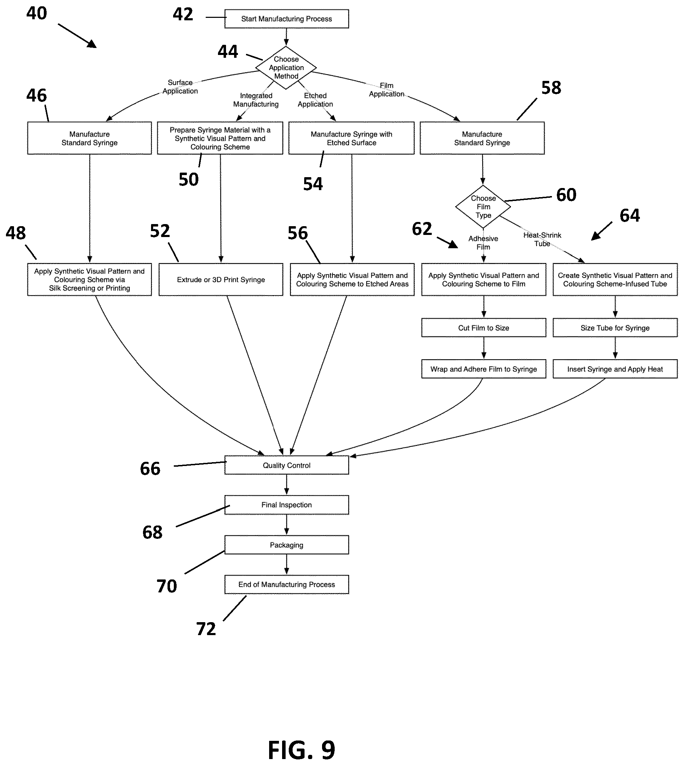

In FIG. 8, the barrel 12 is provided with the half-barrel design 28 and the plunger 16 is provided with the plunger design 26. The plunger flange 18 is also provided with the plunger flange indicia 30, and the barrel flange 14 is also provided with the barrel flange indicia 32. As is stated above with respect to FIG. 6 and 7, while a colour is used for the indicia 30, 32, a design could be alternatively or additionally used on the flanges 14, 18. Turning now to FIG. 9, a flowchart illustrates an exemplary manufacturing process for syringes according to the present invention, including different application paths (surface application, integrated manufacturing, etched application, film application) and decision points.

The process 40 begins with a Start Manufacturing Process step 42. The next step 44 is where the syringe producer chooses a visual indicia application method. There are four main options: surface application (where the design and/or colour are applied to the syringe exterior after manufacturing of the syringe has been completed), integrated manufacturing (where the design and/or colour is incorporated into the syringe during the manufacturing process), etched application (where the surface of the manufactured syringe is etched to produce the design and/or colour), and film application (where the design and/or colour is applied to the surface of the manufactured syringe via a prepared film).

If the surface application method is selected at step 44, step 46 involves manufacturing of the syringe using any known method suitable for manufacturing a syringe that can have the visual indicia applied to its surface. At step 48, the visual indica is applied to the outer surface of the syringe using silk screening, printing, or analogous techniques. The syringe with visual indicia then moves to quality control at step 66 and final inspection at step 68, followed by packaging at step 70 and the end of the manufacturing process at step 72.

If the integrated manufacturing method is selected at step 44, step 50 involves preparing the syringe base material as well as the visual indicia material (design and/or colour), and then extruding, mold injecting, or 3D printing (or using an analogous technique) the syringe concurrently with the visual indicia at the appropriate stage of manufacture as would be known to those skilled in the art. The syringe with visual indicia then moves to quality control at step 66 and final inspection at step 68, followed by packaging at step 70 and the end of the manufacturing process at step 72.

If the etched application method is selected at step 44, step 54 involves manufacturing the syringe with an etched surface, and at step 56 applying the design and/or colour to the etched surface. The syringe with visual indicia then moves to quality control at step 66 and final inspection at step 68, followed by packaging at step 70 and the end of the manufacturing process at step 72.

If the film application method is selected at step 44, the syringe is manufactured using conventional methods at step 58. At step 60 a decision is required as to which film type will be used - adhesive film or a heat-shrink tube. If the adhesive film type is chosen, the method continues at step 62 to applying the design and/or colour to the film, cutting the film to the appropriate dimensions, and wrapping the film around the syringe and adhering it thereto, followed by quality control at step 66 and final inspection at step 68, followed by packaging at step 70 and the end of the manufacturing process at step 72. If the heat-shrink tube type is chosen, the method continues at step 64 to creating a design and/or colour infused tube, sizing the tube for the syringe, and then inserting the syringe into the tube and applying heat, followed by quality control at step 66 and final inspection at step 68, followed by packaging at step 70 and the end of the manufacturing process at step 72. While discarding of spent syringes is a recognized and widespread problem, and has accordingly been discussed above, it will now be clear to those skilled in the art that embodiments of the present invention may be advantageous in other settings including health care institutions and laboratory settings. Even though such institutional settings often present non-natural work areas, suitable visual indicia including vibrant colours could help personnel detect the presence of a spent syringe that has not yet been properly disposed of.

The foregoing is considered as illustrative only of the principles of the present invention. The scope of the claims should not be limited by the exemplary embodiments set forth in the foregoing but should be given the broadest interpretation consistent with the specification as a whole.

## Claims

1. A syringe (10) comprising:
a barrel (12);
a plunger (16) configured for retention within the barrel; and
at least one of the barrel (12) and the plunger (16) provided with visual indicia (24, 26, 28, 30, 32), the visual indicia (24, 26, 28, 30, 32) comprising one or both of a non-natural design and an enhanced-visibility colour non-obscuring of any manufacturer or syringe content markings on the syringe (10);
such that when the syringe (10) is spent and disposed of in a natural environment, the visual indicia (24, 26, 28, 30, 32) provide a visual contrast to the natural environment to enhance visibility and detectability of the syringe (10).

2. The syringe (10) of claim 1 wherein the visual indicia is selected from the group consisting of a pattern, a colour, and a combination thereof.

3. The syringe (10) of any of claims 1 or 2 wherein the visibility and detectability is with respect to a nearby person, a camera, or an Al detection algorithm.

4. The syringe (10) of any of the preceding claims wherein the visual indicia is positioned non-overlapping to manufacturer or syringe content markings on the syringe (10) or wherein the visual indicia is semi-transparent or comprises a transparent portion and is overlapping with manufacturer or syringe content markings on the syringe (10), so that the manufacturer or syringe content markings are visible therethrough.

5. The syringe (10) of any of the preceding claims wherein the visual indicia is applied to both the barrel (12) of the syringe (10) and the plunger (16) of the syringe (10), and/or wherein the barrel (12) comprises a barrel flange (14) and the visual indicia is applied to the barrel flange (14), and/or wherein the plunger (16) comprises a plunger flange (18) and the visual indicia is applied to the plunger flange (18).

6. The syringe of any of the preceding claims wherein the visual indicia is applied to one or more external surfaces of the syringe (10) after manufacture of the syringe (10), in particular by adhesive film, heat-shrinkable tubing, etching, or printing.

7. The syringe (10) of any of the preceding claims wherein the visual indicia is inherent with the syringe (10) and produced during manufacture of the syringe, in particular wherein the visual indicia is co-extruded with syringe material at the time of manufacture of the syringe (10).

8. The syringe (10) of any of the preceding claims wherein the non-natural design is repeating or non-repeating.

9. The syringe (10) of any of the preceding claims wherein the enhanced-visibility colour is selected from the group consisting of a vivid translucent colour, a retro-reflective colour, and a fluorescent colour.

10. Use of visual indicia (24, 26, 28, 30, 32) on a spent syringe (10) for enhanced visibility and detectability of the spent syringe in a natural environment, the visual indicia (24, 26, 28, 30, 32) comprising one or both of a non-natural design and an enhanced-visibility colour non-obscuring of any manufacturer or syringe content markings on the spent syringe (10).

11. The use of claim 10 wherein the visibility and detectability is with respect to a nearby person, a camera, or an Al detection algorithm.

12. The use of any of claims 10 or 11 wherein the visual indicia is semi-transparent or comprises a transparent portion and is overlapping with manufacturer or syringe content markings on the spent syringe (10), so that the manufacturer or syringe content markings are visible therethrough and/or wherein the enhanced-visibility colour is selected from the group consisting of a vivid translucent colour, a retro-reflective colour, and a fluorescent colour.

13. The use of any of the claims 10 to 12 wherein at least one of the barrel (12) and the plunger (16) comprises a flange (16, 18) and the visual indicia is applied to the flange (16, 18).

14. A method for enhancing visibility and detectability of a syringe (10) in a natural environment, the method comprising the steps of:
a. assessing the natural environment to identify at least one of a natural colour and a natural pattern present in the natural environment;
b. determining at least one non-natural visual indicia;
c. selecting at least one of the non-natural visual indicia as presenting a visual contrast to the natural colour or the natural pattern; and
d. applying the selected non-natural visual indicia to the syringe (10);
such that when the syringe (10) is present in the natural environment, the syringe (10) has enhanced visibility and detectability compared to the surrounding natural environment.

15. The method of claim 14 wherein the non-natural visual indicia comprises at least one of a non-natural colour and a non-natural pattern, in particular wherein the non-natural colour is selected from the group consisting of a vivid translucent colour, a retro-reflective colour, and a fluorescent colour, and/or wherein the enhanced visibility and detectability is with respect to a nearby person, a camera, or an Al detection algorithm.
